# EUROPEAN PATENT APPLICATION

(11) **EP 4 653 062 A1**
(43) Date of publication of application: **26.11.2025**
(21) Application number: 24907857.7
(22) Date of filing: 28.11.2024
(51) Int. Cl.: A63B 24/00, A63B 71/06

(54) **EXERCISE SYSTEM USING HEART RATE INFORMATION**

(30) Priority: 18.12.2023 KR 20230184651
(71) Applicant: Ronfic Co., Ltd., Busan (KR)
(72) Inventor: BAEK, Junyoung, Changwon-si Gyeongsangnam-do 51510 (KR); NOH, Chibeom, Busan 49251 (KR); CHA, Yoorim, Busan 48316 (KR); SHIN, Junsu, Busan 47883 (KR); KIM, Sanghoon, Busan 48262 (KR); BAEK, Dahye, Busan 47550 (KR)
(74) Representative: Berggren Oy
(86) International application number: PCT/KR2024/019159
(87) International publication number: WO 2025/135572

(57) **Abstract**

The present invention provides an exercise performance system using heart rate information, the exercise performance system including: an information entry step in which a user's basic information is entered and usage guidance is provided; an exercise selection step in which it is implemented by the entry of the information in the information entry step and a desired exercise menu is selected by the user; a heart rate connection step in which heart rate information is connected to identify heart rate information before exercise selected in the exercise selection step begins; an exercise performance step in which the exercise selected in the exercise selection step is performed once a heart rate status has been connected in the heart rate connection step; and an exercise result step in which the results of the exercise performed in the exercise performance step are determined.

## Description

### Technical Field

The present invention relates to an exercise performance system in which an exercise course is recommended and selected, and more particularly, to an exercise performance system using heart rate information, in which a user's information is entered, an exercise menu is recommended based on the entered user's information, an exercise course optimized for the user is set, a heart rate measurement device is worn on the user before the performance of the exercise course, heart rate information is determined in real time during the performance of the exercise course and then exercise is performed, rest and stretching are provided upon completion of the exercise, and calories burned, heart rate/step results, and comprehensive comments are provided for the user's performed exercise, thereby enabling exercise optimally personalized for the user.

### Background Art

At present, people today become increasingly interested in their health.

Accordingly, interest in exercise is also growing. While exercise is associated with health, it is also associated with play, allowing people to maintain both health and play purposes.

Regular exercise is essential for a healthy life. While people generally think of exercise as simply a way to strengthen their bodies, its ultimate effects also include improving mental well-being.

Exercise is significantly beneficial not only for physical health but also for mental health. Experts recommend performing exercise at least three times a week for at least 30 minutes at a time.

Such exercise is becoming increasingly popular as fitness these days, and fitness is evolving in conjunction with machines of various technologies.

For example, mobile electronic devices such as MP3 players, other audio players, radios, portable televisions, GPS systems, smart watches, pedometers, and mobile phones that allow users to perform exercise while listening to music are being developed and widely used in conjunction with fitness activities.

Many people who enjoy fitness and exercise use one or more devices to keep themselves entertained, to provide performance data during exercise, and to communicate with others while performing exercise.

With the development of these devices, sophisticated exercise performance monitoring systems are now being provided. Such exercise performance monitoring systems provide easy and convenient access to many physical characteristics related to exercise and fitness activities or other exercise performances, including speed, distance, GPS data, heart rate, pulse rate, blood pressure, and body temperature.

While conventional exercise performance monitoring systems provide a wealth of useful data, they are limited to providing only simple data.

Therefore, there is a growing need for an exercise performance system that provides heart rate information and recommends and selects an exercise menu suitable for the heart rate information.

### Disclosure

### Technical Problem

The present invention has been conceived to overcome the above-described problems, and an object of the present invention is to provide an exercise performance system using heart rate information, in which a user's basic information is entered, user guidance is provided, an exercise menu is recommended based on the entered user's information, either a training process or a quick play process is selected to perform exercise, a heart rate measurement device is worn to measure heart rate information before the performance of exercise, exercise is performed with the heart rate measurement device worn, the heart rate is determined in real time during the performance of the exercise, the heart rate status is categorized based on five stages based on preset conditions, and calories burned, heart rate/step results, and comprehensive comments are provided for the user's performed exercise, thereby enabling exercise optimally personalized for the user.

The objects of the present disclosure are not limited to the object mentioned above, and other objects not mentioned may be clearly understood based on the following description.

### Technical Solution

In order to accomplish the above object, there is provided an exercise performance system using heart rate information, the exercise performance system including: an information entry step in which a user's basic information is entered and usage guidance is provided; an exercise selection step in which it is implemented by the entry of the information in the information entry step and a desired exercise menu is selected by the user; a heart rate connection step in which heart rate information is connected to identify heart rate information before exercise selected in the exercise selection step begins; an exercise performance step in which the exercise selected in the exercise selection step is performed once a heart rate status has been connected in the heart rate connection step; and an exercise result step in which the results of the exercise performed in the exercise performance step are determined.

Alternatively, the exercise selection step includes: a training process in which exercise and rest periods are repeated; and a quick play process in which the intensity of exercise is adjusted but exercise is freely performed.

Alternatively, the heart rate status connected in the heart rate connection step is categorized into multiple zones based on preset conditions and operates in conjunction with the exercise performance step to monitor the heart rate status in real time during exercise.

Alternatively, the training process is divided into two types, and includes a primary exercise sequence which involves exercise at maximum speed followed by complete rest and a secondary exercise sequence which involves exercise at fast speed followed by stretching rest.

Alternatively, the quick play process involves multiple exercise intensity stages, which are set by the user as desired.

### Advantageous Effects

The present invention based on the above configuration may be expected to achieve the following effects:
The basic information of a user is entered, and a user guidance process may be provided.

The user guidance process may be performed only upon initial connection to the exercise performing system using heart rate information according to the present invention.

Furthermore, the user guidance process may be implemented and performed when the user wants it.

Once the basic information has been entered and the exercise performing system using heart rate information according to the present invention has been connected, the user may select either the training process, which involves repeating exercise and rest periods, or the quick play process, which involves adjusting the intensity of exercise and performing exercise for a preset duration.

A single process is selected and exercise suitable for the process is performed. The training process is the process of repeating performing exercise for a preset time, followed by rest, up to nine times. The quick play process is the process of performing exercise at a user-selected intensity for a preset time and then terminating the exercise. This allows the user to select and perform exercise suitable for the user.

It may be possible to obtain the heart rate information of the user in real time while exercise is performed.

The heart rate status is categorized into five stages. The user may obtain his or her current heart rate information, and may change his or her behavioral state based on the heart rate information.

After the exercise course has been completed, the user may check his exercise results.

In this case, the exercise results include calories burned, heart rate/step comprehensive comments, exercise time, target heart rate, heart rate entry time, and steps.

### Description of Drawings

FIG. 1 is a diagram of the overall configuration of an exercise performance system using heart rate information according to a preferred embodiment of the present invention;
FIG. 2 is a flowchart of the overall sequence of the exercise performance system using heart rate information according to a preferred embodiment of the present invention;
FIG. 3 is a flowchart of the training performance process of the exercise performance system using heart rate information according to a preferred embodiment of the present invention; and
FIG. 4 is a flowchart of the quick play performance process of the exercise performance system using heart rate information according to a preferred embodiment of the present invention.

### Mode for Invention

An exercise performance system using heart rate information according to a preferred embodiment of the present invention will be described below with reference to the accompanying drawings.

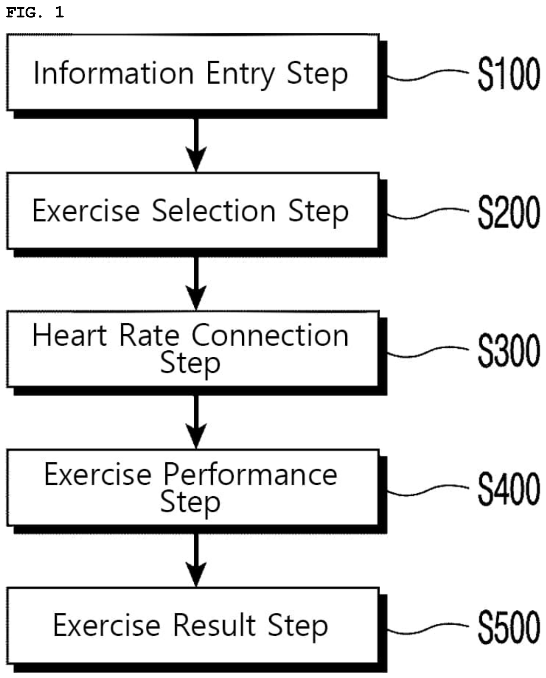

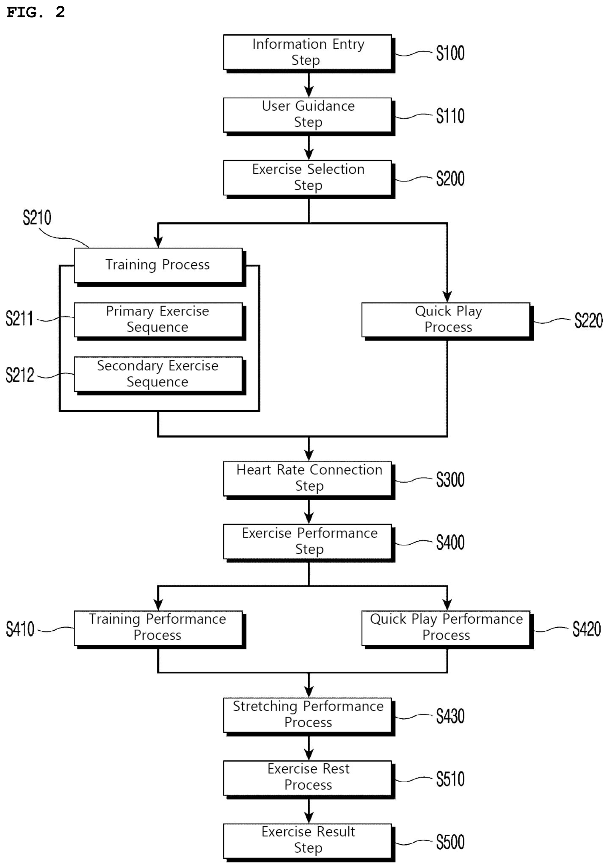

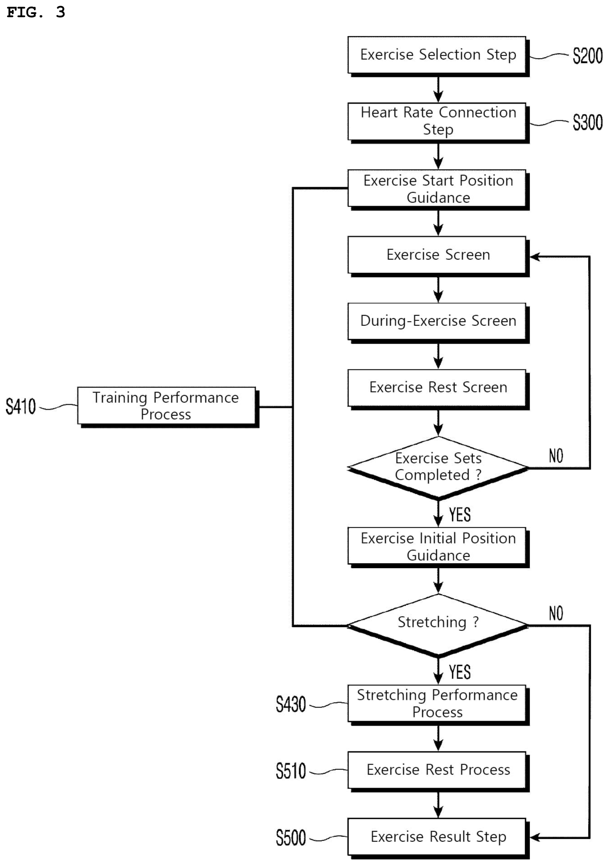

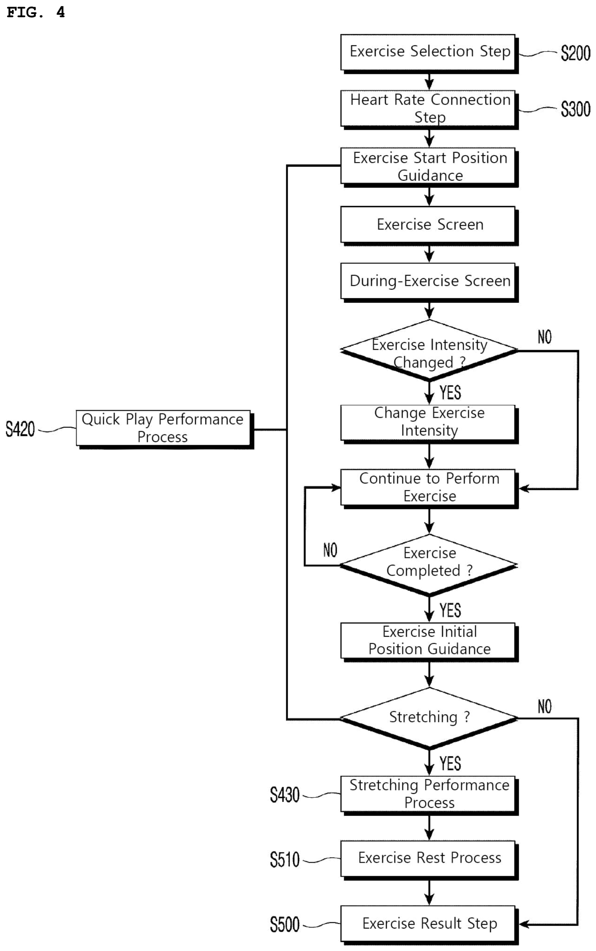

FIG. 1 is a diagram of the overall configuration of an exercise performance system using heart rate information according to a preferred embodiment of the present invention, FIG. 2 is a flowchart of the overall sequence of the exercise performance system using heart rate information according to a preferred embodiment of the present invention, FIG. 3 is a flowchart of the training performance process of the exercise performance system using heart rate information according to a preferred embodiment of the present invention, and FIG. 4 is a flowchart of the quick play performance process of the exercise performance system using heart rate information according to a preferred embodiment of the present invention.

The system according to a preferred embodiment of the present invention may include an information entry step S100, an exercise selection step S200, a heart rate connection step S300, an exercise performance step S400, and an exercise result step S500, as shown in FIG. 1.

First, the information entry step S100 will be discussed.

A user may access the exercise machine program system of the present invention by using a communication-enabled device, such as a communication device, a portable communication device, or the like.

A user needs to register for membership when accessing the present invention for the first time. An existing user may log in by using his or her existing ID and password to access and use the system.

Registration for membership may allow the entry of the basic information of the user.

More specifically, a user's name, date of birth, gender, contact information, and nickname may be entered upon registration for membership.

Upon successful initial login after registration for membership, a user guidance process S110 may be performed.

More specifically, the user guidance process S110 may be performed upon initial successful login after registration for membership, and may provide basic knowledge and usage guidance on the exercise selection step S200, the heart rate connection step S300, the exercise performance step S400, and the exercise result step S500, which will be described later.

In this case, when the user guidance process S110 is in progress and does not need to be continued, it may be canceled by the user's choice, and the exercise selection step S200 may be performed.

Furthermore, when required by a user who does not log in for the first time, the user guidance process S110 may be performed and provide basic knowledge and guidance on the exercise selection step S200, the heart rate connection step S300, the exercise performance step S400, and the exercise result step S500.

Next, the exercise selection step S200 will be discussed.

After registration for membership and logging in in the information entry step S100, the exercise selection step S200 may be performed.

In this case, before the exercise selection step S200 starts, a central part (not shown) may be provided first and allow icons to be selected.

The central part may allow the exercise selection step S200 to be performed, and may support system updates, system errors, and system initialization.

More specifically, when there is an update, the system update may be performed by checking for the latest version before accessing the information entry step S100. The process may proceed to the central part, an update icon may be selected and implemented, and the update may be performed.

When the latest version update is not performed, the exercise selection step S200 will not be performed. Only after the update is performed may the exercise selection step S200 be performed.

Furthermore, a function in which, when an abnormal termination occurs during the use of the exercise performance system using heart rate information according to the present invention, an error is detected, is reported, and is corrected through initialization may be supported.

By selecting the icon for the exercise selection step S200 on the central part, the exercise selection step S200 may be implemented and performed.

The exercise selection step S200 may include a training process S210 in which exercise and rest periods are repeated and a quick play process S220 in which the intensity of exercise is adjusted by a user for a preset time.

The user may select either the training process S210 or the quick play process S220 depending on the purpose of the exercise.

More specifically, the user may select the training process S210 when the user's purpose of the exercise is revving-up, circuit fartlek, and long slow distance running, and may select the quick play process S220 when more flexible exercise is required.

The training process S210 may include two types: a primary exercise sequence S211 which involves exercise at maximum speed followed by complete rest, and a secondary exercise sequence S212 which involves exercise at fast speed followed by stretching rest.

More specifically, the primary exercise sequence S211 may involve 30 to 60 seconds of maximum speed exercise followed by 10 to 30 seconds of complete rest, and the secondary exercise sequence S212 may involve 1 minute 30 seconds to 2 minutes 30 seconds of fast speed exercise followed by 20 to 40 seconds of stretching rest.

The primary exercise sequence S211 and the secondary exercise sequence S212 may be automatically set to a mixed mode or an independent mode according to the user's set intensity of exercise.

The quick play process S220 may allow the user to perform exercise on his or her own without any specific purpose of exercise while adjusting the intensity of exercise resistance on his or her own.

More specifically, the quick play process S220 may allow the user to freely set the intensity of exercise and the step height, allowing for exercise without time constraints.

Furthermore, exercise time may be measured only for the user's actual exercise time, with rest time not being counted as exercise time.

Next, the heart rate connection step S300 will be discussed.

An exercise mode may be selected in the exercise selection step S200, and a heart rate status may be connected to identify heart rate information before exercise.

More specifically, an exercise mode is selected in the exercise selection step S200, and a heart rate monitor connection screen appears before exercise begins. In this case, the user's heart rate information may be checked.

In this case, a heart rate measurement device is not limited, but it may be preferable to use a device including a heart rate sensor.

More specifically, the heart rate measurement device includes electrocardiography, photoplethysmography (PPG), a sphygmomanometer method, and phonocardiography.

The heart rate measurement device may be composed of a pulse sensor and utilize photoplethysmography to measure heart rate information.

Photoplethysmography is divided into transmissive and reflective types. In the transmissive-type method, infrared and red light is radiated onto the surface of the human body, and changes in blood flow caused by the heartbeat are measured by changes in the amount of light transmitted through the body, enabling the measurement of heart rate.

In the reflective-type method, infrared and red light with a green wavelength of around 550 nm is radiated onto the living body, and a photodiode or phototransistor is used and measures the light reflected inside the living body. Since arterial blood contains oxygenated hemoglobin, which has the characteristic of absorbing incident light, it is possible to measure the changes in blood flow caused by the heartbeat in a time series.

When the heart rate measurement device is worn on the user, the heart rate is measured. However, when the heart rate is not measured, a heart rate connection failure message may be provided.

The heart rate connection failure message may be provided when a connection is not established for 30 seconds, and may be provided until the connection is established.

In this case, it may be possible for the user to exclude the heart rate connection step S300 when heart rate information is not required.

More specifically, the heart rate connection step S300 may be excluded in situations where heart rate information is not available, such as a situation where the user does not need heart rate information during exercise and a situation where the heart rate measurement device is not installed.

When the heart rate connection step S300 is excluded, the heart rate information is provided as a default value of 60. The intensity of exercise shown every one to two minutes is determined, so that the heart rate information is predicted and determined.

Once a heart rate measurement device has been worn and a resting heart rate has been identified, exercise may be performed in the exercise performance step S400, which will be described below.

Next, the exercise performance step S400 will be discussed.

The exercise performance step S400 enables exercise to be performed in the exercise mode selected in the exercise selection step S200.

More specifically, in the exercise performance step S400, the training performance process S410 is performed when the training process S210 is selected as the exercise mode selected in the exercise selection step S200, and the quick play performance process S420 is performed when the quick play process S220 is selected as the exercise mode.

In the training performance process S410, the primary exercise sequence S211 involving exercise at maximum speed for 30 to 60 seconds followed by complete rest for 10 to 30 seconds may be set as one set, and the secondary exercise sequence S212 involving exercise at fast speed for 1 minute 30 seconds to 2 minutes 30 seconds followed by stretching rest for 20 to 40 seconds may be set as one set.

The training execution process S410 may enable exercise to be performed with the primary exercise sequence S211 or the secondary exercise sequence S212 set to desired sets.

In this case, as for desired sets, programs may be preset as shown in Table 1 below, a desired program may be selected, and then exercise may be performed.

**Table 1**

| Category | Exercise Time (1 set) | Rest Time (1 set) | Total Sets |
|---|---|---|---|
| Primary Exercise Sequence | 30 to 60 seconds | 10 to 30 seconds | 4 sets |
| | | | 8 sets |
| | | | 12 sets |
| | | | 18 sets |
| Secondary Exercise Sequence | 1 minute 30 seconds to 2 minutes 30 seconds | 20 to 40 seconds | 4 sets |
| | | | 6 sets |
| | | | 9 sets |
| | | | 12 sets |

As shown in Table 1, a desired program may be preset, allowing the user to select the desired program with preset values and perform exercise.

The quick play performance process S420 allows the user to adjust the intensity and steps of exercise. In this case, the intensity of exercise is adjusted at a total of five levels, and exercise is performed.

In the quick play performance process S420, default exercise time is set to 10 minutes. The intensity of exercise may be changed at any time during the exercise. When the exercise time exceeds 10 minutes and the user wants to continue the exercise, the user may continue the exercise.

During the training performance process S410 and the quick play performance process S420, it may be possible to identify the heart rate status, connected during the heart rate connection step S300, in real time while the user is performing the exercise.

The heart rate may be categorized into five zones depending on the heart rate status, as shown in Table 2 below. The time available for exercise may be determined based on the corresponding zone.

**Table 2**

| Zone | %HRₘₐₓ | Effect | Available Time |
|---|---|---|---|
| Zone 1 | 50 to 60 | Warm-up | 20 to 40 minutes |
| Zone 2 | 60 to 70 | Light aerobic exercise | 40 to 80 minutes |
| Zone 3 | 70 to 80 | Improvement in cardiovascular efficiency | 10 to 40 minutes |
| Zone 4 | 80 to 90 | Cardiorespiratory endurance training | 2 to 10 minutes |
| Zone 5 | 90 to 100 | Maximum capacity | Less than 5 minutes |

As shown in Table 2, the effect and the available time are recommended based on the user's heart rate. The user may perform exercise based on the recommended effect and available time.

In either the training performance process S410 or the quick play performance process S420, it may be possible for the system to be initialized when the user arbitrarily terminates the exercise during the performance of the exercise.

Furthermore, when the exercise is terminated in any way, as in the case where the exercise is terminated arbitrarily or the exercise is completed and thus terminated, it may be possible to select whether to proceed with the stretching performance process S430.

The stretching performance process S430 is a process in which guidance on stretching is provided to the user after the exercise is completed. The stretching performance process S430 may be performed when necessary, and may not be performed when not necessary.

When stretching is completed, it may be possible to check the results of the exercise.

Next, the exercise result step S500 will be discussed.

In the exercise result stage S500, it may be possible to check the results of the exercise performed in the exercise performance step S400.

In this case, it may be possible for the user's heart rate to recover and muscles to recover during the exercise rest process S510 before the results are checked in the exercise result step S500.

The exercise rest process S510 is the step which follows the completion of the exercise performance step S400 and during which the heart rate recovers. In this step, deep breathing is performed very slowly for two minutes to allow the heart rate to recover.

When the heart rate does not recover after 2 minutes, the exercise will resume. Once the heart rate recovers, the exercise results may be checked.

The results of the exercise may include exercise time, calories burned, comprehensive comments on heart rate/steps, target heart rate, and heart rate entry time.

The exercise time is marked as actual exercise time and rest time during exercise, and the calories burned may be marked as food names associated with the calories burned.

The target heart rate is calculated and marked based on the THR. The heart rate entry time may be marked in such a manner that exercise time is marked for individual heart rate zones so that the exercise time is categorized and marked for individual heart rate zones within the exercise time.

As for the steps, the number of steps for the exercise performed is marked, and the number of steps may be the same as the number of steps marked during the exercise.

Once the exercise results have been checked, "Terminate" is selected, and the exercise selection step S200 may be implemented and resumed.

Based on the above-described configuration, the operational state of the present invention will be discussed.

The user may access the exercise performance system using heart rate information according to the present invention by using a communication device or a mobile communication device.

Upon initial login, the user performs a process of registration for membership at the information entry step S100, where basic information may be entered.

After completing the registration for membership, the user guidance process S110 may be performed upon logging in for the first time.

The provision of guidance on basic knowledge and instructions is completed by the performance of the user guidance process S110, and the exercise selection step S200 may be performed.

The exercise selection step S200 allows the user to choose between the training process S210, which alternates between exercise and rest periods, and the quick play process S220, which allows the user to perform exercise without time constraints but with the intensity of exercise adjustable by the user.

When the user's purpose of exercise is to improve basic physical strength, perform diet, or strengthen muscles, the training process S210 may be selected. In contrast, when the user's purpose of exercise is condition adjustment, interval training, circuit fartlek, and low-intensity long slow distance running, the quick play process S220 may be selected.

Once the exercise mode has been selected, the heart rate status may be connected in the heart rate connection step S300.

In the heart rate connection step S300, a heart rate measurement device is worn on the user's body, and the user's resting heart rate may be determined.

In this case, when the user does not require heart rate information, the heart rate connection step S300 is excluded. The heart rate information is provided at a default value of 60, and the intensity of exercise is checked every 1 to 2 minutes. This allows for the heart rate to be predicted and determined.

Once heart rate information has been connected in the heart rate connection step S300, exercise may be performed in the exercise mode selected in the exercise selection step S200.

When the training process S210 is selected in the exercise selection step S200, the training performance process S410 will be performed. In contrast, when the quick play process S220 is selected in the exercise selection step S200, the quick play performance process S420 will be performed.

The exercise is performed in such a manner that exercise and rest are performed in preset periods. It may be possible to select a preset program and thus perform exercise in such a manner that the exercise is performed for a number of sets suitable for the user.

When exercise is performed, the heart rate status is identified. The heart rate is identified based on five zones. It may be possible to check the time available for exercise according to the corresponding zone.

Once the performance of the exercise is completed, guidance on the stretching performance process S430 is provided, thereby allowing the user to be guided through a stretching process.

Once stretching has been completed, the exercise rest process S510 is performed, and thus the heart rate is recovered, and the exercise result step S500 is performed, and thus the results of the exercise are determined.

The results of the exercise may include exercise time, calories burned, comprehensive comments on heart rate/steps, target heart rate, and heart rate entry time.

Therefore, the true technical protection scope of the present invention should encompass not only the above-described embodiments but also various modifications based on the technical spirit of the present invention defined in the claims to be described later.

## Claims

1. An exercise performance system using heart rate information, the exercise performance system comprising:
an information entry step in which a user's basic information is entered and usage guidance is provided;
an exercise selection step in which it is implemented by entry of the information in the information entry step and a desired exercise menu is selected by the user;
a heart rate connection step in which heart rate information is connected to identify heart rate information before exercise selected in the exercise selection step begins;
an exercise performance step in which the exercise selected in the exercise selection step is performed once a heart rate status has been connected in the heart rate connection step; and
an exercise result step in which results of the exercise performed in the exercise performance step are determined.

2. The exercise performance system of claim 1, wherein the exercise selection step comprises:
a training process in which exercise and rest periods are repeated; and
a quick play process in which intensity of exercise is adjusted but exercise is freely performed.

3. The exercise performance system of claim 1, wherein the heart rate status connected in the heart rate connection step is categorized into multiple zones based on preset conditions and operates in conjunction with the exercise performance step to monitor the heart rate status in real time during exercise.

4. The exercise performance system of claim 2, wherein the training process is divided into two types, and comprises:
a primary exercise sequence which involves exercise at maximum speed followed by complete rest; and
a secondary exercise sequence which involves exercise at fast speed followed by stretching rest.

5. The exercise performance system of claim 2, wherein the quick play process involves multiple exercise intensity stages, which are set by the user as desired.
